# EUROPEAN PATENT SPECIFICATION

(11) **EP 4 076 562 B1**
(45) Date of publication and mention of the grant of the patent: **12.08.2026**
(21) Application number: 20839504.6
(22) Date of filing: 16.12.2020
(51) Int. Cl.: A61L 29/16, A61M 25/00

(54) **MEDICAL PRODUCTS INCLUDING SILVER ANTIMICROBIAL, HYDRATION MEDIUM AND A STABILIZING AGENT**
MEDIZINISCHE PRODUKTE, DIE EIN ANTIMIKROBIELLES FEUCHTIGKEITSSPENDENDES MEDIUM UND EIN STABILISIERUNGSMITTEL ENTHALTEN
PRODUITS MÉDICAUX COMPRENANT UN AGENT ANTIMICROBIEN À BASE D'ARGENT, UN MILIEU D'HYDRATATION ET UN AGENT DE STABILISATION

(30) Priority: 20.12.2019 US 201962951740 P
(43) Date of publication of application: 26.10.2022
(73) Proprietor: Hollister Incorporated, Libertyville, IL 60048 (US)
(72) Inventor: SILEIKA, Tadas, S., Libertyville, IL 60048 (US); DEAN, Nicole, L., Libertyville, IL 60048 (US); PETTIGREW, Jacqueline, MJ, Libertyville, IL 60048 (US)
(74) Representative: FRKelly
(86) International application number: PCT/US2020/065403
(87) International publication number: WO 2021/127040

(56) References cited:
- WO-A1-2014/093056
- WO-A2-2005/107455
- US-B1- 6 605 751

## Description

### TECHNICAL FIELD

The present disclosure generally relates to packaged medical products that contain a medical device having a silver antimicrobial, a hydration medium and a stabilizing agent, such as a masking agent, wherein the stabilizing agent reduces discoloration of the hydration medium caused by silver.

### BACKGROUND

Some medical devices are required to be hydrated with a hydration medium or stored in a hydration medium, such as liquid water or saline. Such devices may include hydrophilic urinary catheters, contact lenses, etc. These medical devices may be packaged in a package containing the medical device and the hydration medium. In some instances, it may be desirable for the medical device to include a silver antimicrobial. One challenge with including a silver antimicrobial is when the device is packaged in contact with the hydration medium is that the silver or other components of the antimicrobial may cause the hydration medium to become discolored. For example, a clear hydration medium may become reddish from the silver antimicrobial. An example of a packaged urinary catheter comprising a catheter comprising a hydrophilic polymer with antimicrobial silver and a hydration liquid is shown in WO 2014/093056 A1.

When the package is opened, there is a risk that the hydration medium unintentionally spills or leaks from the package. If the discolored hydration medium contacts the user's clothing, the hydration medium may leave a stain on the fabric of the clothing. Furthermore, the user may find a reddish hydration medium unsightly.

Therefore, there remains a need for packaged medical devices that include antimicrobials, a hydration medium and reduces the risk of the hydration medium staining the user's clothes.

### SUMMARY

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter product that includes a urinary catheter including a catheter tube and a lubricous coating disposed on the catheter tube, the lubricous coating comprising a hydrophilic polymer. A silver antimicrobial is within the coating. The product also includes a hydration medium including a liquid. The product further includes a stabilization agent that is a Chloride or Bromide salt, or calcium halide that interacts with one or more components of the silver antimicrobial to reduce discoloration of the hydration medium from the silver antimicrobial, wherein when the stabilization agent is the calcium halide the silver antimicrobial comprises iodine.

### BRIEF DESCRIPTION OF FIGURES

Fig. 1 is a plan view of a medical device product in accordance with the present disclosure; and
Fig. 2 is cross-sectional view of the medical device of Fig. 1.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

The present disclosure discloses urinary catheter products that include a silver antimicrobial, a hydration medium, and a stabilizing agent, such as a masking agent. The stabilizing agent may be associated with the urinary catheter or the hydration medium. The urinary catheter may be any urinary catheter that utilizes a silver antimicrobial. The silver antimicrobial is in a hydrophilic material of the device. The hydrophilic material, such as hydrophilic coatings, includes the silver antimicrobial therein. The hydrophilic material may be a hydrophilic polymer. The silver antimicrobial may be bonded to the polymer or may be in free form within the polymer matrix. The silver antimicrobial may be at the surface of a polymer coating or may be impregnated within or blended with the polymer coating. Furthermore, the silver antimicrobial may be uniform throughout the coating or the coating may have selected portions that have more silver antimicrobial than others.

The urinary catheter products also include a hydration medium. The hydration medium may be any suitable hydration medium for hydrating and/or storing the urinary catheter. The hydration medium may include a liquid, such as water, and any suitable additives or agents.

The medical product also includes a stabilizing agent, such as a masking agent, that reduces discoloration of the hydration medium which may result from the silver antimicrobial or components thereof. The stabilizing agent may also enhance the antimicrobial effect. The stabilizing agent may be in the hydration fluid or be associated with the urinary catheter.

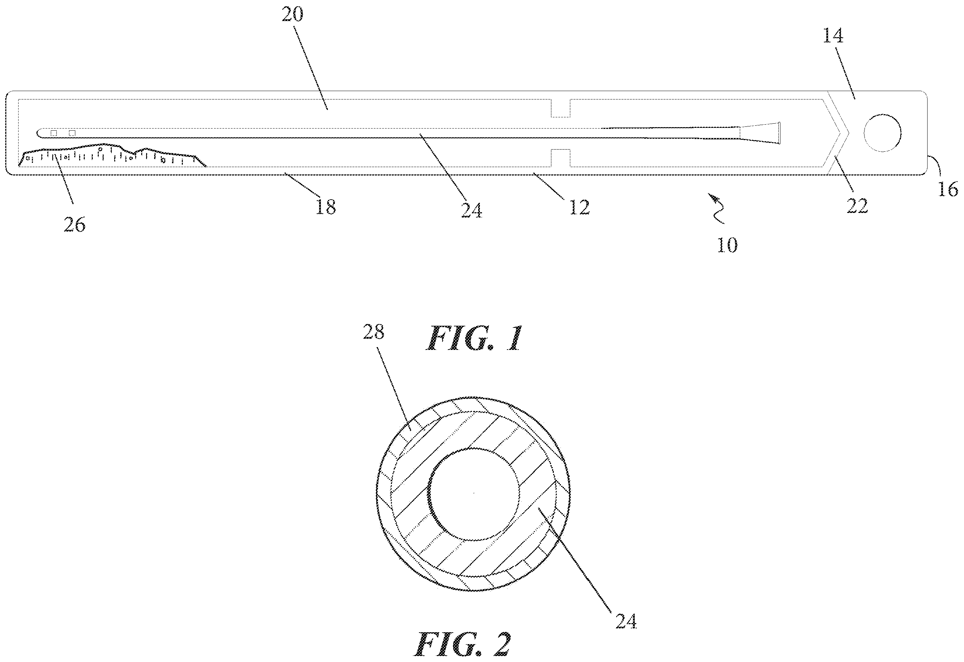

Fig. 1 shows one exemplary medical device product 10. In this figure, the medical device is illustrated as a urinary catheter 24. For example, the urinary catheter may be at least partially made from a hydrophilic material and may be packaged with a hydration medium 26, such as liquid water or saline. The packaged hydrophilic urinary catheter product 10 includes a package 12 that surrounds that catheter 24. In the embodiment illustrated in Fig. 1, the package 12 is formed from a front sheet 14 and back sheet (not shown) that are sealed together to form a peripheral seal 18 and define an internal cavity 20. At the top of the package 12, the front and back sheets may be unattached above top seal 22. The package 12 may be opened by grasping these unattached portions and pulling the front sheet 14 and back sheet away from each other to peal open the package 12 along seal 18. Optionally, the package 12 may be any other suitable package for containing a urinary catheter. For example, the package may be a tear open package. Additionally, the material of the package, optionally, may be made from a gas impermeable material.

A hydrophilic urinary catheter is contained within cavity 20. As mentioned above, in the illustrated embodiment, the hydrophilic urinary catheter is a hydrophilic catheter 24. The hydrophilic catheter 24 may be any suitable hydrophilic catheter that includes a hydrophilic outer surface that becomes lubricous when hydrated with a hydration medium, such as water. For example and referring to Fig. 2, the catheter 24 may include a lubricious hydrophilic coating 28 on the outer surface of the catheter. The hydrophilic coating includes a hydrophilic polymer and a silver antimicrobial within the hydrophilic polymer. The silver antimicrobial may be a silver salt, such as a salt of silver and iodine. In one alternative, the silver antimicrobial may be silver periodate, such as pentasilver hexaoxoiodate (Ag₅IO₆).

A hydration medium, such as a hydration liquid 26, also may be located within the cavity 20. The hydration medium may be loose or free flowing within the cavity 20, or the hydration medium may be in a compartment within the cavity 20 wherein the compartment keeps the hydration medium stored separately from the catheter 24. During manufacturing or just prior to use, the separate compartment may be opened so that the hydration medium 26 is free flowing with the cavity 20 and contacts the catheter. The hydration medium may be water or saline that includes any suitable additives.

The product may also include a stabilizing agent. The stabilizing agent may be in the hydration medium and/or within the hydrophilic coating. The stabilizing agent is a salt that includes a chloride, bromide, phosphate, sulfate, or fluoride anion that binds with silver. The salt may be, for example, magnesium chloride or barium bromide. Furthermore, the stabilizing agent may include a cation that binds with iodine, such as one or more calcium halides. In one embodiment, the stabilizing agent may be calcium chloride. In other embodiments the stabilizing may be calcium phosphate, calcium sulfate, calcium fluoride, calcium bromide. When the stabilizing agent, such as calcium chloride or any of the other suitable salt, is within the hydration medium, the stabilizing agent may be in an amount of 0.0001 wt% to 10 wt% of the hydration medium, preferably about 1 wt%. The stabilizing agent may also serve to increase the antimicrobial performance of the device.

When catheter 24 is stored in contact with hydration medium, silver and/or iodine ions (when silver iodine is used) may migrate from the catheter and into the hydration medium. When the stabilizing agent is calcium chloride, free silver ions bind with the chloride ions from calcium chloride, precipitating as a white solute into the coating. A transient amount of free silver ions remains in solution, without metallization (via reduction) into metallic silver, which otherwise would cause the discoloration. Similarly, when iodide is present, the iodide ions form a salt with the calcium ions from calcium chloride, creating colorless salt. Otherwise, free iodine would cause an amber color to emerge for the solution.

It will be understood that the embodiments described above are illustrative of some of the applications of the principles of the present subject matter. Numerous modifications may be made by those skilled in the art without departing from the scope of the claimed subject matter. For these reasons, the scope hereof is not limited to the above description but is as set forth in the following claims.

## Claims

1. A urinary catheter product (10), comprising:
a package (12) having a cavity (20) containing:
a urinary catheter (24) at least partially made from a hydrophilic polymer and a silver antimicrobial within the hydrophilic polymer;
a hydration medium (26) loose within the cavity (20) of the package (12), the hydration medium (26) including a liquid in contact with the urinary catheter (24); and
a stabilization agent is a chloride, or bromide salt, or calcium halide that interacts with one or more components of the silver antimicrobial to reduce discoloration of the hydration medium (26) from the silver antimicrobial, wherein when the stabilization agent is the calcium halide the silver antimicrobial comprises iodine.

2. The product of claim 1, wherein the hydration medium (26) includes the stabilization agent.

3. The product of any one of claims 1 and 2, wherein the stabilization agent is within the hydrophilic polymer.

4. The product of any one of claims 1-3, wherein the urinary catheter (24) includes a catheter tube and the hydrophilic polymer comprises a lubricous coating disposed on the catheter tube.

5. The product of any one of claims 1-4, wherein the silver antimicrobial comprises a silver salt.

6. The product of any one of claims 1-5, wherein the silver antimicrobial comprises silver and iodine.

7. The product of any one of claims 1-6, wherein the silver antimicrobial comprises silver periodate.

8. The product of any one of claims 1-7, wherein the silver antimicrobial comprises pentasilver hexaoxoiodate (Ag₅IO₆).

9. The product of any one of claims 1-8, wherein the stabilization agent comprises an anion that binds with silver.

10. The product of any one of claims 1-9, wherein the stabilization agent comprises a cation that binds with iodine.

11. The product of any one of claims 1-10, wherein the stabilization agent comprises calcium chloride.

12. The product of claim 2, wherein the stabilization agent comprises calcium chloride in an amount of 0.0001 wt% to 10 wt% of the hydration medium, preferably about 1 wt%.

13. The product of claim 12, wherein the hydration medium (24) includes other additives.

## Patentansprüche

1. Harnkatheterprodukt (10), umfassend:
eine Verpackung (12) mit einem Hohlraum (20), enthaltend:
einen Harnkatheter (24), der mindestens teilweise aus einem hydrophilen Polymer und einem Silber-Antimikrobiotikum innerhalb des hydrophilen Polymers hergestellt ist;
ein feuchtigkeitsspendendes Medium (26) lose innerhalb des Hohlraums (20) der Verpackung (12), wobei das feuchtigkeitsspendende Medium (26) eine mit dem Harnkatheter (24) in Kontakt stehende Flüssigkeit beinhaltet; und
einen Stabilisator als ein Chlorid- oder Bromidsalz oder Calciumhalogenid, das mit einer oder mehreren Komponenten des Silber-Antimikrobiotikums interagiert, um eine Verfärbung des feuchtigkeitsspendenden Mediums (26) durch das Silber-Antimikrobiotikum zu verhindern, wobei das Silber-Antimikrobiotikum Iod umfasst, wenn es sich bei dem Stabilisator um das Calciumhalogenid handelt.

2. Produkt nach Anspruch 1, wobei das feuchtigkeitsspendende Medium (26) den Stabilisator beinhaltet.

3. Produkt nach einem der Ansprüche 1 und 2, wobei sich der Stabilisator innerhalb des hydrophilen Polymers befindet.

4. Produkt nach einem der Ansprüche 1 bis 3, wobei der Harnkatheter (24) einen Katheterschlauch beinhaltet und das hydrophile Polymer eine auf dem Katheterschlauch angeordnete schlüpfrige Beschichtung umfasst.

5. Produkt nach einem der Ansprüche 1 bis 4, wobei das Silber-Antimikrobiotikum ein Silbersalz umfasst.

6. Produkt nach einem der Ansprüche 1 bis 5, wobei das Silber-Antimikrobiotikum Silber und Iod umfasst.

7. Produkt nach einem der Ansprüche 1 bis 6, wobei das Silber-Antimikrobiotikum Silberperiodat umfasst.

8. Produkt nach einem der Ansprüche 1 bis 7, wobei das Silber-Antimikrobiotikum Pentasilberhexaoxoiodat (Ag₅IO₆) umfasst.

9. Produkt nach einem der Ansprüche 1 bis 8, wobei der Stabilisator ein an Silber bindendes Anion umfasst.

10. Produkt nach einem der Ansprüche 1 bis 9, wobei der Stabilisator ein an Iod bindendes Kation umfasst.

11. Produkt nach einem der Ansprüche 1 bis 10, wobei der Stabilisator Calciumchlorid umfasst.

12. Produkt nach Anspruch 2, wobei der Stabilisator Calciumchlorid in einer Menge von 0,0001 Gew.-% bis 10 Gew.-% des feuchtigkeitsspendenden Mediums, vorzugsweise etwa 1 Gew.-%, umfasst.

13. Produkt nach Anspruch 12, wobei das feuchtigkeitsspendende Medium (24) andere Zusätze beinhaltet.

## Revendications

1. Produit de cathéter urinaire (10), comprenant :
un emballage (12) présentant une cavité (20) contenant :
un cathéter urinaire (24) constitué au moins partiellement d'un polymère hydrophile et d'un agent antimicrobien à base d'argent au sein du polymère hydrophile ;
un milieu d'hydratation (26) libre à l'intérieur de la cavité (20) de l'emballage (12), le milieu d'hydratation (26) comportant un liquide en contact avec le cathéter urinaire (24) ; et
un agent de stabilisation qui est un sel de chlorure ou de bromure, ou un halogénure de calcium qui interagit avec un ou plusieurs composants de l'agent antimicrobien à base d'argent pour réduire la décoloration du milieu d'hydratation (26) provoquée par l'agent antimicrobien à base d'argent, dans lequel, lorsque l'agent de stabilisation est l'halogénure de calcium, l'agent antimicrobien à base d'argent comprend de l'iode.

2. Produit selon la revendication 1, dans lequel le milieu d'hydratation (26) comporte l'agent de stabilisation.

3. Produit selon l'une quelconque des revendications 1 et 2, dans lequel l'agent de stabilisation se trouve au sein du polymère hydrophile.

4. Produit selon l'une quelconque des revendications 1 à 3, dans lequel le cathéter urinaire (24) comporte un tube de cathéter et le polymère hydrophile comprend un revêtement lubrifiant disposé sur le tube de cathéter.

5. Produit selon l'une quelconque des revendications 1 à 4, dans lequel l'agent antimicrobien à base d'argent comprend un sel d'argent.

6. Produit selon l'une quelconque des revendications 1 à 5, dans lequel l'agent antimicrobien à base d'argent comprend de l'argent et de l'iode.

7. Produit selon l'une quelconque des revendications 1 à 6, dans lequel l'agent antimicrobien à base d'argent comprend du périodate d'argent.

8. Produit selon l'une quelconque des revendications 1 à 7, dans lequel l'agent antimicrobien à base d'argent comprend de l'hexaoxoiodate de pentaargent (Ag₅IO₆).

9. Produit selon l'une quelconque des revendications 1 à 8, dans lequel l'agent de stabilisation comprend un anion qui se lie à l'argent.

10. Produit selon l'une quelconque des revendications 1 à 9, dans lequel l'agent de stabilisation comprend un cation qui se lie à l'iode.

11. Produit selon l'une quelconque des revendications 1 à 10, dans lequel l'agent de stabilisation comprend du chlorure de calcium.

12. Produit selon la revendication 2, dans lequel l'agent de stabilisation comprend du chlorure de calcium en une quantité allant de 0,0001 % en poids à 10 % en poids du milieu d'hydratation, de préférence d'environ 1 % en poids.

13. Produit selon la revendication 12, dans lequel le milieu d'hydratation (24) comporte d'autres additifs.
